(19) Europäisches Patentamt
European Patent Office
Office européen des brevets

(11) Veröffentlichungsnummer: **0 469 357 A1**

(12) # EUROPÄISCHE PATENTANMELDUNG

(21) Anmeldenummer: **91111537.6**

(22) Anmeldetag: **11.07.91**

(51) Int. Cl.[5]: **A01N 43/56**, C07D 401/04, C07D 403/04

(30) Priorität: **24.07.90 DE 4023488**

(43) Veröffentlichungstag der Anmeldung: **05.02.92 Patentblatt 92/06**

(84) Benannte Vertragsstaaten: **AT CH DE DK FR GB IT LI SE**

(71) Anmelder: **BAYER AG**
**W-5090 Leverkusen 1 Bayerwerk(DE)**

(72) Erfinder: **Sasse, Klaus, Dr.**
**Pützweg 13**
**W-5060 Bergisch Gladbach 2(DE)**
Erfinder: **Schwamborn, Michael Dr.**
**Prämonstratenserstrasse 76**
**W-5000 Köln 80(DE)**
Erfinder: **Wachtler, Peter, Dr.**
**Morgengraben 4**
**W-5000 Köln 80(DE)**
Erfinder: **Frie, Monika, Dr.**
**Im Alten Driesch 1**
**W-5068 Odenthal(DE)**
Erfinder: **Ludwig, Georg-Wilhelm, Dr.**
**Bethelstrasse 22**
**W-4150 Krefeld 1(DE)**
Erfinder: **Paulus, Wilhelm, Dr.**
**Deswatinesstrasse 90**
**W-4150 Krefeld 1(DE)**
Erfinder: **Schmitt, Hans-Georg, Dr.**
**Am Oberend 13**
**W-4150 Krefeld 1(DE)**

(54) **1-[Pyri(mi)dyl-(2)]-5-hydroxy-pyrazol-Mikrobizide.**

(57) Die Erfindung betrifft die Verwendung bestimmter, durch die in der Beschreibung angegebene Formel (I) charakterisierte 1-[Pyri(mi)dyl-(2)]-5-hydroxy-pyrazole als Mikrobizide für den Schutz technischer Materialien und bestimmte neue, durch die in der Beschreibung angegebene Formel (II) charakterisierte 1-[Pyri(mi)dyl-(2)]-5-hydroxy-pyrazole.

EP 0 469 357 A1

Die Erfindung betrifft die Verwendung bestimmter 1-[Pyri(mi)dyl-(2)]-5-hydroxy-pyrazole als Mikrobizide für den Schutz technischer Materialien und bestimmte neue 1-[Pyri(mi)dyl-(2)]-5-hydroxy-pyrazole.

Aus der US-A 4 663 327 sind 1-Hetaryl-4-aryl-pyrazolin-5-one, z.B. 1-[Pyri(mi)dyl-(2)]-4-phenyl-pyrazolin-5-one und deren mikrobizide Eigenschaften bekannt. Da die Verbindungen jedoch farbig sind und bei ihrer Einarbeitung in technische Materialien, z.B. Anstrichmittel und Kunststoffe, zu Verfärbungen führen, sind sie trotz ihrer guten mikrobiziden Eigenschaften im Materialschutz nicht verwendbar.

In der veröffentlichten japanischen Anmeldung JA-A 62/149 617 (CA Vol. 108, 11 247 k) sind ferner 1-Hetaryl-pyrazolin-5-one, z.B. 1-[Pyri(mi)dyl-(2)]-pyrazolin-5-one und ihre Verwendung als Pharmazeutika beschrieben.

Bislang werden zur fungiziden Ausrüstung von Kunststoffen noch arsenorganische Verbindungen eingesetzt, obgleich der Ersatz dieser Verbindungen und auch der noch in Anstrichmitteln verwendeten quecksilberorganischen Verbindungen aus ökotoxikologischen Gründen sehr erwünscht ist. Bislang wurden jedoch noch keine ökotoxikologisch günstigeren Verbindungen gefunden, die die hohen Anforderungen erfüllen, die an für die mikrobizide Ausrüstung von Kunststoffen verwendbaren Mikrobizide gestellt werden müssen. Solche Mikrobizide müssen nämlich zusätzlich zu einer guten mikrobiziden Wirksamkeit auch noch eine hohe Temperaturbeständigkeit aufweisen und dürfen außerdem die Eigenschaften der Kunststoffe nicht beeinträchtigen.

Es wurde jetzt gefunden, daß 1-[Pyri(mi)dyl-(2)]-5-hydroxy-pyrazole einer bestimmten Formel in Kunststoffen und Anstrichmitteln eine mikrobizide Wirkung aufweisen, die der der arsenorganischen und quecksilberorganischen Verbindungen gleichkommt und die außerdem die erforderliche thermische Stabilität besitzen und die weiterhin die Bedingungen erfüllen, ökotoxikologisch unbedenklich zu sein und die Eigenschaften, z.B. Farbe und Elastizität der mit ihnen ausgerüsteten Kunststoffe, Anstrichmittel und Anstriche nicht zu beeinträchtigen.

Die Erfindung betrifft daher die Verwendung von 1-[Pyri(mi)dyl-(2)]-5-hydroxy-pyrazolen der Formel

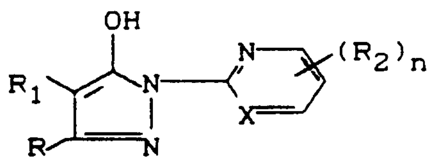 (I),

in der

- R für Wasserstoff, oder eine gegebenenfalls substituierte Alkyl-, Aralkyl- oder Alkoxygruppe steht,
- $R_1$ Wasserstoff, eine gegebenenfalls substituierte Alkyl-, Alkenyl-, Alkinyl-, Cycloalkyl-, Aralkyl-, Alkoxy-, Alkylmercapto-, Aralkoxy-, Aralkylmercapto-, Aryloxy-, Arylmercapto-, Alkoxycarbonyl- oder Aminocarbonylgruppe steht, oder $R_1$ zusammen mit R einen zweiwertigen, 3 bis 6 C-Atome in der Kette aufweisenden Alk(en)ylen-Rest bildet,
- $R_2$ für Halogen, Nitro, Cyan oder eine gegebenenfalls substituierte Alkyl-, Alkoxy-, Alkylmercapto-, Alkoxycarbonyl- oder Aminocarbonylgruppe steht,
- n Null oder eine ganze Zahl von 1 bis 3 bedeutet, wobei $R_2$ für gleiche oder verschiedene Reste stehen kann, wenn n 2 oder 3 ist und
- X für CH oder N steht,

als Mikrobizide zum Schutz technischer Materialien.

Bevorzugt werden solche 1-[Pyri(mi)dyl-(2)]-5-hydroxy-pyrazole der Formel (I) verwendet, in der

- X die unter Formel (I) angegebene Bedeutung hat,
- n Null ist und
- R für Wasserstoff, Alkyl oder Aralkyl und
- $R_1$ für Wasserstoff, gegebenenfalls substituiertes Alkyl, Cycloalkyl, Aralkyl, Alkoxy, Aralkylmercapto oder Alkoxycarbonyl stehen.

Besonders bevorzugt werden solche 1-[Pyri(mi)dyl-(2)]-5-hydroxy-pyrazole der Formel (I), in der

- X die unter Formel (I) angegebene Bedeutung hat,
- n Null ist und
- R für Wasserstoff, $C_6$-$C_{12}$-Alkyl oder Benzyl und
- $R_1$ für Wasserstoff, $C_1$-$C_8$-Alkyl, $C_5$-$C_7$-Cycloalkyl, $C_1$-$C_6$-Alkoxy, gegebenenfalls substituiertes Benzyl, $C_1$-$C_6$-Alkoxycarbonyl oder Benzylmercapto stehen.

Ganz besonders bevorzugt sind die 1-[Pyri(mi)dyl-(2)]-5-hydroxy-pyrazole der Formel (I), wenn in dieser

- X für CH steht,

n Null ist und
R Wasserstoff und
$R_1$ $C_1$-$C_4$-Alkyl, Benzyl, Chlorbenzyl oder Ethoxycarbonyl bedeuten.

Von den erfindungsgemäß zu verwendenden 1-[Pyri(mi)dyl-(2)]-5-hydroxy-pyrazolen sind die Verbindungen der Formel (II) neu.

Die Erfindung betrifft daher auch neue 1-[Pyri(mi)dyl]-5-hydroxy-pyrazole der Formel

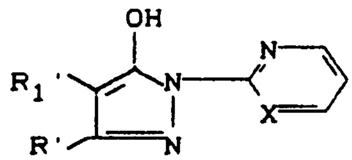

(II),

in der

X für CH oder N steht,
R' für Wasserstoff, oder eine gegebenenfalls substituierte $C_6$-$C_{10}$-Alkyl-, Aralkyl- oder Alkoxygruppe steht,
$R_1$' Wasserstoff, eine gegebenenfalls substituierte $C_6$-$C_{12}$-Alkyl-, Alkenyl-, Alkinyl-, Cycloalkyl-, Aralkyl-, Alkoxy-, Alkylmercapto-, Aralkoxy-, Aralkylmercapto-, Aryloxy-, Arylmercapto-, Alkoxycarbonyl-oder Aminocarbonylgruppe steht, oder $R_1$' zusammen mit R' einen zweiwertigen 3 bis 6 C-Atome in der Kette aufweisenden Alk(en)ylen-Rest bildet,

mit der Maßgabe, daß R' und $R_1$' nicht gleichzeitig für Wasserstoff stehen.

Typische Vertreter der erfindungsgemäß verwendbaren Pyrazole der Formel (I) und der erfindungsgemäßen Pyrazole der Formel (II) sind in den Beispielen aufgeführt.

Die erfindungsgemäß zu verwendenden Pyrazole der Formel (I) und die erfindungsgemäßen neuen Pyrazole der Formel (II) können auch in ihrer tautomeren Pyrazolin-5-on-Form vorliegen.

Als gegebenenfalls substituierte Alkylreste seien für R und $R_1$ genannt: $C_1$-$C_{12}$-Alkylgruppen wie Methyl, Ethyl, n- und i-Propyl, n-Butyl, i-Pentyl, Hexyl, i-Octyl, n-Decyl und n-Dodecyl; als Substituenten kommen für diese Alkylreste vor allem Halogenatome, vorzugsweise Chlor oder Fluor, ferner $C_1$-$C_4$-Alkoxy und $C_1$-$C_6$-Alkoxycarbonylgruppen in Betracht. Vertreter der substituierten Alkylreste sind beispielsweise der Difluormethyl-, Trifluormethyl-und der Monochlordifluormethylrest, ferner der Methoxymethyl-und der Methoxycarbonylmethyl-und der Ethoxycarbonylmethylrest.

Für R, $R_1$, R' und $R_1$' seien genannt:

Als gegebenenfalls substituierte Aralkylgruppen der Benzyl-, α-Methylbenzyl-, α,α-Dimethylbenzyl-, 2-Phenylethyl- und der α- und β-Naphthylmethyl-Rest und durch Halogen, insbesondere Chlor und Fluor, $C_1$-$C_4$-Alkyl, insbesondere Methyl, Ethyl, Trifluormethyl, Difluorchlormethyl, Difluormethyl und Trichlormethyl, durch $C_1$-$C_4$-Alkoxy, insbesondere Methoxy, Ethoxy, Trifluormethoxy, Difluorchlormethoxy und Difluormethoxy, $C_1$-$C_4$-Alkylmercapto wie Methylmercapto, Trifluormethylmercapto, Difluorchlormethylmercapto, Nitro und/oder Cyano substituierte Benzylreste.

Als gegebenenfalls substituierte Alkoxygruppen:

$C_1$-$C_{12}$-Alkoxygruppen wie die Methoxy, Ethoxy, Butoxy und Hexoxygruppe.

Für $R_1$ und $R_1$' seien genannt:

Als gegebenenfalls substituierte Alkenylgruppe die Allylgruppe;

als gegebenenfalls substituierte Alkinylgruppen die Propinyl-, 1-Iod-propinyl- und die 3,3-Dimethylpropinylgruppe;

als gegebenenfalls substituierte Cycloalkylgruppen $C_5$-$C_7$-Cycloalkylgruppen, insbesondere durch $C_1$-$C_4$-Alkylgruppen oder Halogen substituierte Cyclohexylreste wie der Cyclohexylrest, der Methylcyclohexyl-, der Dimethylcyclohexyl- und der 1,3,3-Trimethylcyclohexyl- und der 3-Chlorcyclohexylrest;

als gegebenenfalls substituierte Alkylmercaptogruppen durch Halogen substituierte $C_1$-$C_2$-Alkylmercaptogruppen wie die Methylmercapto-, Trifluormethylmercapto-, Difluormethylmercapto- und die Difluorchlormethylmercaptogruppe;

als Aralkoxygruppen die Benzyloxygruppe und durch Halogen und $C_1$-$C_4$-Alkylreste substituierte Benzyloxygruppen;

als Aralkylmercaptogruppen die Benzylmercaptogruppe und die durch Halogen und/oder $C_1$-$C_4$-Alkyl substituierten Benzylmercaptogruppen;

als Aryloxygruppen die Phenoxygruppe und durch Halogen, $C_1$-$C_4$-Alkyl, $C_1$-$C_2$-Halogenalkyl, Cyan, Nitro,

Trifluormethyl und Difluormethyl substituierte Phenoxygruppen;
als Arylmercaptogruppen die Phenylmercaptogruppe und die durch Halogen, $C_1$-$C_4$-Alkyl, $C_1$-$C_2$-Halogenalkyl, Cyan und Nitro substituierte Phenylmercaptogruppe;
als Alkoxycarbonylgruppen $C_1$-$C_6$-Alkoxycarbonylgruppen wie die Methoxycarbonyl- und Ethoxycarbonylgruppe;
als Aminocarbonylgruppe die Carbonamid-, N-Methylcarbonamid- und N,N-Dimethylaminocarbonamidgruppe;
als 3 bis 6 C-Atome in der Kette aufweisende Alk(en)ylen-Reste der Ethylen-, Propylen-1,3-, Butylen-1,4- und der Butadien-(1,4)ylen-1,4-Rest.

Für $R_2$ seien als gegebenenfalls substituierte Alkylreste $C_1$-$C_4$-Alkyl-, $C_1$-$C_2$-Halogenalkylreste wie der Methyl-, Trifluormethyl-, Difluormethyl- und Difluorchlormethylrest genannt.

Die erfindungsgemäß zu verwendenden Pyrazole der Formel (I) und die erfindungsgemäßen neuen Pyrazoline der Formel (II) sind nach an sich bekannten, z.B. in den Chem. Ber. 38 (1905) 2104; Bull. Soc. Chim. France 1967, 3780-92 beschriebenen Kondensationsreaktionen herstellbar. Diese Kondensationsreaktionen verlaufen nach folgendem Reaktionsschema:

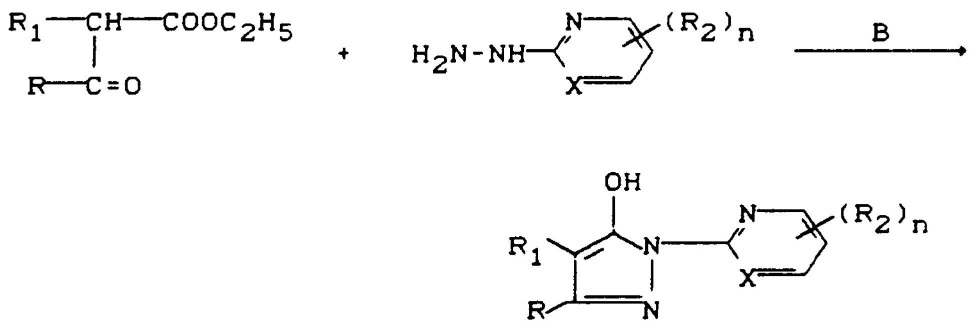

Die Kondensation kann gegebenenfalls in Gegenwart eines Lösungsmittels durchgeführt werden; als Lösungsmittel haben sich vor allem Alkohole wie Ethanol oder aromatische Kohlenwasserstoffe wie Toluol bewährt.

Zur Erleichterung der Ringschlußreaktion setzt man vorteilhafterweise Basen wie Natriumhydroxid, Kaliumhydroxid oder Kalium-tert.-butylat zu.

Die Kondensation kann innerhalb eines größeren Temperaturbereichs ausgeführt werden. Für die zuerst ablaufende Hydrazonbildung kann bei Temperaturen von 20 bis 110 °C gearbeitet werden, vorzugsweise zwischen 60 und 90 °C. Die nach der Basenzugabe ablaufende Cyclokondensationsreaktion kann bei Temperaturen von 20 bis 100 °, vorzugsweise 20 bis 40 °C vorgenommen werden. Da die Basenzugabe in manchen Fällen exotherm verläuft, kann ein Kühlen bei diesem Reaktionsschritt erforderlich sein.

Die 1-[Pyri(mi)dyl]-(2)]-5-hydroxy-pyrazole werden nach bekannten Methoden aus den Reaktionsgemischen isoliert. Man geht im allgemeinen so vor, daß man die Reaktionsgemische vom Lösungsmittel befreit und den Rückstand mit wäßriger Salzsäure behandelt. Die dabei ausfallenden Pyrazole werden durch Absaugen abgetrennt. Es ist jedoch auch möglich, das Reaktionsgemisch unmittelbar in einen großen Überschuß verdünnter Salzsäure einzugießen und die als Niederschlag sich abscheidenden Pyrazole abzufiltrieren.

Die für die Kondensationsreaktion benötigten Ausgangsverbindungen, gegebenenfalls in $\alpha$-Stellung substituierte $\beta$-Ketocarbonsäureester oder $\alpha$-Formylcarbonsäureester und 2-Hydrazinopyri(mi)dine, sind entweder bekannte Verbindungen oder in Analogie zu bekannten Verbindungen nach vorbeschriebenen Verfahren herstellbar.

Bei den erfindungsgemäß zu schützenden technischen Materialien handelt es sich um nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel, Kunststoffe und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, deren Funktion durch Vermehrung von Mikroorganismen beeinträchtigt werden kann. Im Rahmen der vorliegenden Erfindung seien als technische Materialien vorzugsweise Anstrichmittel, Kunststoffe und Kunststoffartikel genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze sowie Holz dauerhaft verfärbende und holzzerstörende Pilze.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet können die erfindungsgemäß zu verwendenden Wirkstoffe in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Die zum Schutz technischer Materialien verwendeten mikrobiziden Mittel enthalten die Wirkstoffe im allgemeinen in einer Menge von 1 bis 100 Gew.-%, bevorzugt von 10 bis 90 Gew.-%.

Die Anwendungskonzentrationen der erfindungsgemäß zu verwendenden Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäß zu verwendenden Wirkstoffe können auch in Mischung mit anderen bekannten Wirkstoffen angewendet werden. Beispielsweise seien die folgenden Wirkstoffe genannt: Benzylalkoholmono(poly)hemiformal und andere Formaldehyd abspaltende Verbindungen, Benzimidazolylmethylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate, wie 2-Phenyl-phenol, (2,2'-Dihydroxy-5,5'-dichlor)-diphenylmethan und 3-Methyl-4-chlor-phenol, Organo-Zinnverbindungen, N-Trihalogenmethylthio-verbindungen, wie Folpet, Fluorfolpet und Dichlofluanid, Azolfungizide wie Triadimefon, Triadimenol und Bitertanol, Tebuconazole, Propiconazole und Prochloraz sowie Iodpropargylverbindungen wie Iodpropargylbutyl-carbamat.

Darüber hinaus können die erfindungsgemäßen Wirkstoffe, insbesondere die der aufgeführten Beispiele, in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u.a.. Bevorzugte Mischungspartner sind:

1. aus der Gruppe der Phosphorsäureester

Azinphos-ethyl, Azinphos-methyl, 1-(4-Chlorphenyl)-4-(O-ethyl, S-propyl)phosphoryloxypyrazol (TIA-230), Chlorpyrifos, Coumaphos, Demeton, Demeton-S-methyl, Diazinon, Dichlorvos, Dimethoat, Ethoprophos, Etrimfos, Fenitrothion, Fenthion, Heptenophos, Parathion, Parathion-methyl, Phosalon, Pirimiphos-ethyl, Pirimiphos-methyl, Profenofos, Prothiofos, Sulprofos, Triazophos, Trichlorphon.

2. aus der Gruppe der Carbamate

Aldicarb, Bendiocarb, BPMC (2-(1-Methylpropyl)phenylmethylcarbamat), Butocarboxim, Butoxicarboxim,

Carbaryl, Carbofuran, Carbosulfan, Cloethocarb, Isoprocarb, Methomyl, Oxamyl, Pirimicarb, Promecarb, Propoxur, Thiodicarb.

3. aus der Gruppe der Carbonsäureester

Allethrin, Alphamethrin, Bioallethrin, Bioresmethrin, Cycloprothrin, Cyfluthrin, Cyhalothrin, Cypermethrin, Deltamethrin, 2,2-Dimethyl-3-(2-chlor-2-trifluormethylvinyl)cyclopropancarbonsäure-(alpha-cyano-3-phenyl-2-methyl-benzyl)ester (FMC 54800), Fenpropathrin, Fenfluthrin, Fenvalerat, Flucythrinate, Flumethrin, Fluvalinate, Permethrin, Resmethrin, Tralomethrin.

4. aus der Gruppe der Formamidine

Amitraz, Chlordimeform.

5. aus der Gruppe der Zinnverbindungen

Azocyclotin, Cyhexatin, Fenbutatinoxid.

6. Sonstige

Abamektin, Bacillus thuringiensis, Bensultap, Binapacryl, Bromopropylate, Buprofecin, Camphechlor, Cartap, Chlorbenzilate, Chlorfluazuron, 2-(4-Chlorphenyl)-4,5-diphenylthiophen (UBI-T 930), Chlofentezine, Cyclopropancarbonsäure(2-naphthylmethyl)ester (Ro 12-0470), Cyromacin, DDT, Dicofol, N-(3,5-Dichlor-4-(1,1,2,2-tetrafluorethoxy)phenylamino)carbonyl)-2,6-difluorbenzamide (XRD 473), Diflubenzuron, N-(2,3-Dihydro-3-methyl-1,3-thiazol-2-ylidene)2,4-xylidine, Dinobuton, Dinocap, Endosulfan, Fenoxycarb, Fenthiocarb, Flubenzimine, Flufenoxuron, Gamma-HCH, Hexythiazox, Hydramethylnon (AC 217 300), Ivermectin, 2-Nitromethyl-4,5-dihydro-6H-thiazin (SD 52618), 2-Nitromethyl-3,4-dihydrothiazol (SD 35651), 2-Nitromethylene-1,3-thiazinan-3-yl-carbamaldehyde (WL 108 477), Propargite, Teflubenzuron, Tetradifon, Tetrasul, Thiocyclam, Triflumaron, Kernpolyeder- und Granuloseviren.

## Beispiele

### Beispiel 1

Ein Gemisch aus 7,2 g Propionylessigsäureethylester, 5,4 g 2-Hydrazinopyridin und 200 ml Ethanol wird unter Rühren 3 Stunden auf Rückflußtemperatur erwärmt. Dann wird das Reaktionsgemisch auf 20 bis 30° C abgekühlt und unter Rühren mit 5,8 g Kalium-tert.-butylat versetzt. Nach 10 stündigem Rühren wird das Lösungsmittel im Vakuum entfernt, der Rückstand mit 200 ml Wasser versetzt und durch Zugabe von Salzsäure auf einen pH-Wert von 1 eingestellt. Der Niederschlag wird abgesaugt und nach dem Trocknen aus Alkohol umkristallisiert.

Es werden 5,2 g (= 55,1 % der Theorie) 1-[Pyridyl-(2)]-3-ethyl-5-hydroxy-pyrazol erhalten.
Fp: 189° C.

In gleicher Weise wurden aus den entsprechenden Acylessigsäureethylestern die in der nachstehenden Tabelle 1 aufgeführten, in 3-Stellung substituierten 1-[Pyri(mi)dyl-(2)]-5-hydroxy-pyrazole erhalten.

Die in Form von Ölen anfallenden Pyrazole wurden durch ihren durch NMR bestimmten δ-Wert des Pyrazol-H gekennzeichnet.

Tabelle 1

| Beispiel | R ($R_1$, $R_2$ = H) | X | physikalische Kenngrößen |
| --- | --- | --- | --- |
| 2 | $CH_3$ | N | δ = 5,38 ppm |
| 3 | $CF_3$ | CH | δ = 5,85 ppm |
| 4 | t-Butyl | CH | δ = 5,47 ppm |
| 5 | $CH_3OCH_2$ | CH | δ = 5,57 ppm |
| 6 | $iC_3H_7$ | CH | δ = 5,42 ppm |
| 7 | $C_8H_{14}$ | CH | δ = 5,42 ppm |
| 8 | $C_{10}H_{21}$ | CH | δ = 5,42 ppm |
| 9 | 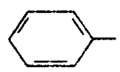 | CH | δ = 5,92 ppm |
| 10 | 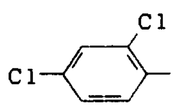 | CH | Fp. = 124-125° C |
| 11 | 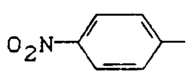 | CH | δ = 5,96 ppm |
| 12 | 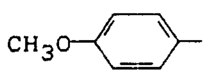 | CH | δ = 6,06 ppm |
| 13 | 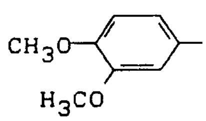 | CH | δ = 6,06 ppm |

Tabelle 1 - Fortsetzung

| Beispiel | R ($R_1$, $R_2$ = H) | X | physikalische Kenngrößen |
|---|---|---|---|
| 14 | 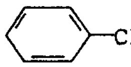 $CH_2$- | CH | δ = 5,32 ppm |
| 15 | $CH_3$- | N | $M^{\oplus}$ = 176 |
| 16 | 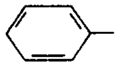 | N | Fp. = 146-49° C |

Beispiel 17

Ein Gemisch aus 24,9 g α-Formyl-octansäureethylester (73 %ig), 9,8 g 2-Hydrazinopyridin und 200 ml Ethanol wird unter Rühren 3 Stunden auf Rückflußtemperatur erhitzt. Dann wird das Reaktionsgemisch auf 20 bis 30° C abgekühlt und unter Rühren portionsweise mit 10,4 g Kalium-tert.-butylat versetzt. Nach 10-stündigem Rühren wird das Lösungsmittel im Vakuum entfernt. Der Rückstand wird mit 200 ml Wasser versetzt und mit Salzsäure auf einen pH-Wert 1 eingestellt. Das sich abscheidende Öl wird mit Methylenchlorid extrahiert. Nach dem Trocknen der Methylenchloridlösung über Natriumsulfat wird das Lösungsmittel im Vakuum entfernt.

Es werden 12,1 g (= 54,8 % der Theorie) 1-[Pyridyl-(2)]-4-hexyl-5-hydroxy-pyrazol in Form eines gelben, langsam kristallisierenden Öles erhalten.

Fp.: 37-38° C

In gleicher Weise wurden aus den entsprechenden α-Formyl-Carbonsäureethylestern die in der nachstehenden Tabelle 2 aufgeführten, in 4-Stellung substituierten 1-[Pyri(mi]dyl-(2)]-5-hydroxy-pyrazole erhalten.

Tabelle 2

| Beispiel | $R_1$ (R, $R_2$ = H) | X | physikalische Kenngrößen |
|---|---|---|---|
| 18 | $CH_3$ | CH | δ = 7,35 ppm |
| 19 | $C_2H_5$ | CH | δ = 7,35 ppm |
| 20 | $i-C_3H_7$ | CH | δ = 7,48 ppm |
| 21 | $n-C_4H_9$ | CH | δ = 7,36 ppm |
| 22 | $C_{10}H_{21}$ | CH | δ = 7,50 ppm |
| 23 | $i-C_5H_{11}$ | CH | δ = 7,52 ppm |
| 24 | $CH_3$ | N | δ = 7,51 ppm |
| 25 | $C_2H_5$ | N | δ = 7,50 ppm |
| 26 | $n-C_4H_9$ | N | δ = 7,41 ppm |
| 27 | 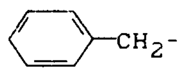 | -CH | δ = 7,35 ppm |
| 28 | 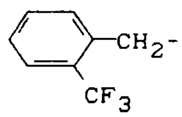 | -CH | δ = 7,33 ppm |
| 29 | 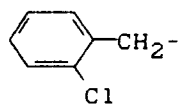 | -CH | δ = 7,30 ppm |
| 30 | 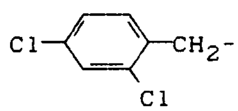 | -CH | δ = 7,28 ppm |

Tabelle 2 - Fortsetzung

| Beispiel | $R_1$ ($R$, $R_2$ = H) | X | physikalische Kenngrößen |
|---|---|---|---|
| 31 | 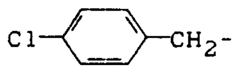 | -CH | δ = 7,32 ppm |
| 32 | 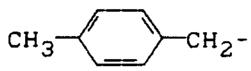 | -CH | δ = 7,33 ppm |
| 33 | 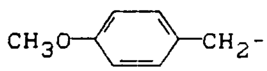 | CH | Fp.: 70-71° C |
| 34 | 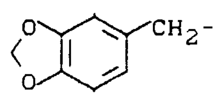 | CH | Fp.: 156-157° C |
| 35 | 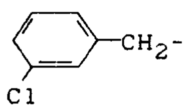 | CH | Fp.: 67-68° C |
| 36 | 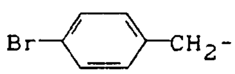 | CH | δ = 7,28 ppm |
| 37 | 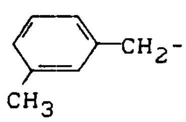 | CH | Fp.: 60-61° C |
| 38 | 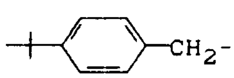 | CH | δ = 7,22 ppm |

Tabelle 2 - Fortsetzung

| Beispiel | $R_1$ (R, $R_2$ = H) | X | physikalische Kenngrößen |
|---|---|---|---|
| 39 | 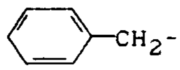 | N | Fp.: 110° C |
| 40 | 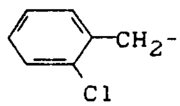 | N | δ = 7,50 ppm |
| 41 | 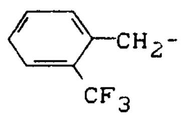 | N | δ = 7,42 ppm |
| 42 | 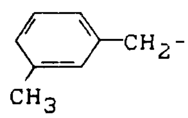 | N | Fp.: 110-12° C |
| 43 | 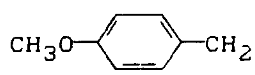 | N | Fp.: 102-103° C |
| 44 | 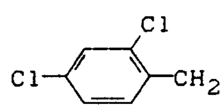 | N | Fp.: 192-94° C |
| 45 | 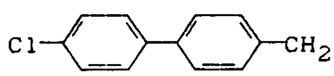 | N | δ = 7,37 ppm |

<u>Tabelle 2</u> - Fortsetzung

| Beispiel | $R_1$ (R, $R_2$ = H) | X | physikalische Kenngrößen |
|---|---|---|---|
| 46 | O<br>‖<br>$CH_3OC-$ | CH | $\delta$ = 7,86 ppm |
| 47 | O<br>‖<br>$C_2H_5OC-$ | CH | $\delta$ = 7,84 ppm |
| 48 | O<br>‖<br>$C_3H_7C-$ | CH | $\delta$ = 7,85 ppm |
| 49 | $t-C_4H_9C-$<br>‖<br>O | CH | $\delta$ = 7,70 ppm |
| 50 | $C_8H_{17}OC-$<br>‖<br>O | CH | $\delta$ = 7,87 ppm |
| 51 | $C_2H_5OCO-$ | N | Fp.: 155-58° C |
| 52 | $C_2H_5OCO-CH_2-$ | C | $\delta$ = 7,61 ppm |
| 53 | $n-C_4H_9OCO-CH_2-$ | C | $\delta$ = 7,50 ppm |
| 54 | $n-C_6H_{13}OCO-CH_2$ | C | $\delta$ = 7,59 ppm |
| 55 | $i-C_4H_9OCO-CH_2$ | C | $\delta$ = 7,86 ppm |

Tabelle 2 - Fortsetzung

| Beispiel | $R_1$ (R, $R_2$ = H) | X | physikalische Kenngrößen |
|---|---|---|---|
| 56 | $C_8H_{17}OCO-CH_2-$ | C | $\delta$ = 7,45 ppm |
| 57 | $C_2H_5NH-CO-CH_2-$ | C | $\delta$ = 7,42 ppm |
| 58 | $C_2H_5OCO-CH_2-$ | N | $\delta$ = 7,62 ppm |
| 59 | $C_6H_{13}-S$ | CH | $\delta$ = 7,30 ppm |
| 60 | 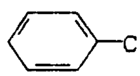 $-CH_2-S$ | CH | $\delta$ = 7,20 ppm |
| 61 | $CH_3O-$ | CH | $\delta$ = 7,38 ppm |
| 62 | $C_4H_9O-$ | CH | $\delta$ = 7,32 ppm |
| 63 | $CH_2=CH-CH_2-$ | CH | $\delta$ = 7,35 ppm |
| 64 | 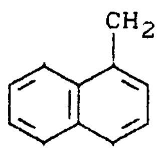 | CH | $\delta$ = 7,47 ppm |

Beispiel 65

Ein Gemisch aus 14,1 g $\alpha$-Benzyl-benzoylessigsäureethylester, 5,5 g Hydrazinopyridin und 100 ml Ethanol wird unter Rühren 3 Stunden auf Rückflußtemperatur erwärmt. Anschließend wird das Reaktionsgemisch auf 20 bis 30° C abgekühlt und unter Rühren portionsweise mit 5,6 g Kalium-tert.-butylat versetzt. Das Reaktionsgemisch wird 15 Stunden bei 20 bis 30° C gerührt. Anschließend wird das Lösungsmittel im Vakuum entfernt. Der Rückstand wird mit 200 ml Wasser versetzt und mit Salzsäure auf einen pH-Wert von 1 eingestellt. Der Niederschlag wird abgesaugt und mit Isopropanol verrührt.

Es werden 10 g (61,1 % der Theorie) 2-[Pyridyl-(2)]-3-phenyl-4-benzyl-5-hydroxy-pyrazol erhalten. Fp.: 115-116° C.

In gleicher Weise wurden aus den entsprechend substituierten Acylessigsäureethylestern die in der folgenden Tabelle 3 angegebenen, in 3- und 4-Stellung substituierten 1-[Pyri(mi)dyl-(2)]-5-hydroxy-pyrazole erhalten.

Tabelle 3

| Beispiel | R | $R_1$ ($R_2$ = H) | X | Fp. (°C) |
|---|---|---|---|---|
| 66 | $CH_3$ | $CH_3$ | CH | 123,5-26° C |
| 67 | $C_2H_5$ | $CH_3$ | CH | 50-53 |
| 68 | i-$C_3H_7$ | $CH_3$ | CH | 71-72 |
| 69 | $CH_3$ | $C_2H_5$ | CH | 107-108 |
| 70 | $CH_3$ | n-$C_4H_9$ | CH | 94-97 |
| 71 | $CH_3$ | n-$C_6H_{13}$ | CH | 42-43 |
| 72 | $CH_3$ | n-$C_7H_{15}$ | CH | 39-40 |
| 73 | $CH_3$ | $C_{12}H_{25}$ | CH | 47-48 |
| 74 | $CH_3$ | 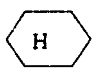 | CH | 82-83 |
| 75 | $CH_3$ | 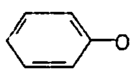-O-$(CH_2)_2$ | CH | 121-124 |
| 76 | $CH_3$ | 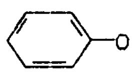-O-$(CH_2)_3$ | CH | 84-87 |
| 77 | $CH_3$ | $HC{\equiv}C-C(CH_3)_2-$ | CH | 57-59 |

Tabelle 3 - Fortsetzung

| Beispiel | R | $R_1$ ($R_2$ = H) | X | Fp. (°C) |
|---|---|---|---|---|
| 78 | $CH_3$ | 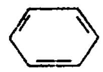 | CH | 114-115 |
| 79 | $CH_3$ | 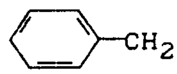 | CH | 56-63 |
| 80 | $CH_3$ | 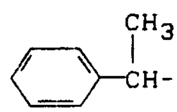 | CH | 62-64 |
| 81 | $CH_3$ | 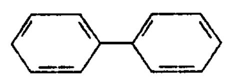 | CH | 153-154 |
| 82 | R +$R_1$ = | $(CH_2)_4$ | CH | 125-126 |
| 83 | R +$R_1$ = | $(CH_2)_5$ | CH | 108-110 |
| 84 | $CH_3$ | $C_2H_5$ | N | 111-113 |
| 85 | $C_2H_5$ | $CH_3$ | N | 109-112 |
| 86 | i-$C_3H_7$ | $CH_3$ | N | 84-86 |
| 87 | $CH_3$ | n-$C_4H_9$ | N | 88-89 |
| 88 | $CH_3$ | n-$C_7H_{15}$ | N | 63-65 |
| 89 | $CH_3$ | n-$C_{12}H_{25}$ | N | 73-75 |

Tabelle 3 - Fortsetzung

| Beispiel | R | $R_1$ ($R_2$ = H) | X | Fp. (°C) |
|---|---|---|---|---|
| 90 | $CH_3$ | 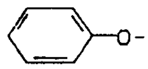 $-O-(CH_2)_2$ | N | 121-124 |
| 91 | $CH_3$ | 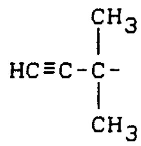 | N | 108-11 |
| 92 | $CH_3$ | 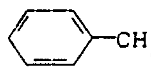 $-CH_2$ | N | 78-83 |
| 93 | 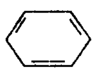 | 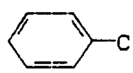 $-CH_2$ | N | 155-61 |
| 94 | $CH_3$ | 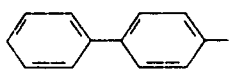 | N | 198-201 |
| 95 | $CH_3$ | 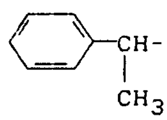 | N | 86-87 |
| 96 | R $+R_1$ = | $(CH_2)_4$ | N | 192-196 |

<u>Tabelle 3</u> Fortsetzung

| Beispiel | $R_1$ | $R_2$ (R = H) | X | Fp. (°C) |
|---|---|---|---|---|
| 97 | $C_2H_5$ | 5-Cl-, 6-$CH_3$-NH-, | N | 172 |
| 98 | $C_2H_5$ | 5-Br | CH | 119 |
| 99 | $C_2H_5$ | 6-$CH_3$ | CH | δ(Pyrazol-H) = 7,37 ppm |
| 100 | $C_6H_{13}$ | 4-$CF_3$, 6-$CH_3$ | CH | δ(Pyrazol-H) = 7,38 ppm |
| 101 | $C_6H_{13}$ | 6-$CH_3$ | CH | δ(Pyrazol-H) = 7,34 ppm |
| 102 | $C_6H_{13}$ | 5-Cl, 6-$CH_3$NH- | N | δ(Pyrazol-H) = 7,45 ppm |
| 103 | $C_8H_{17}$ | 5-Cl | CH | 109 |
| 104 | 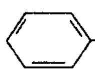 $CH_2$- | 4-Cl, 6-$CH_3$ | CH | 80 |
| 105 | 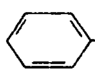 $CH_2$ | 6-$CH_3$ | CH | δ(Pyrazol-H) = 7,31 ppm |
| 106 | 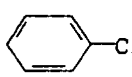 $CH_2$ | 6-Cl | CH | 167-168 |

<u>Tabelle 3</u> - Fortsetzung

| Beispiel | $R_1$ | $R_2$ (R = H) | X | Fp. (°C) |
|---|---|---|---|---|
| 107 | 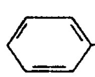 $CH_2$ | 5-Br | CH | 143 |
| 108 | 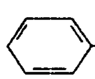 $CH_2$ | 5-$NO_2$ | CH | 170 |

Anwendungsbeispiele

Beispiel A

Zum Nachweis der Wirksamkeit gegen Pilze wurden die minimalen Hemm-Konzentrationen (MHK) der erfindungsgemäßen Wirkstoffe bestimmt:
Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28 °C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt; sie ist in der nachstehenden Tabelle 4 angegeben.

Tabelle 4 MHK Werte mg/l verschiedener 1-[Pyri(mi)dyl-(2)]-5-hydroxy-pyrazole auf Pilze

| Testorganismen | 1-[Pyri(mi)dyl-(2)]-5-hydroxy-pyrazole gemäß Beispiel | | | | | | | | | |
|---|---|---|---|---|---|---|---|---|---|---|
| | 21 | 19 | 26 | 27 | 39 | 40 | 41 | 35 | 31 | 36 |
| Alternaria tenuis | 5 | 50 | 25 | 50 | 20 | 20 | 20 | 35 | 50 | 10 |
| Aspergillus niger | 2 | 10 | 50 | 20 | 50 | 50 | 50 | 20 | 50 | 20 |
| Aureobasidium pullulans | 35 | 50 | 20 | 20 | 10 | 35 | 35 | 50 | 35 | 20 |
| Chaetomium globosum | 5 | 2 | 20 | 50 | 50 | 20 | 20 | 50 | 50 | 50 |
| Cladosporium cladosporioides | 10 | 5 | - | - | - | - | - | - | - | - |
| Cladosporium herbarum | - | - | 50 | 50 | 50 | 15 | 15 | 50 | 35 | 35 |
| Lentinus tigrinus | 1 | 2 | 75 | 50 | 75 | 100 | 100 | 50 | 50 | 50 |
| Penicillium glaucum | 2 | 20 | - | - | - | - | - | - | - | - |
| Penicillium breviante | - | - | 50 | 100 | 50 | 20 | 20 | - | 20 | 20 |
| Sclerophoma pityophila | 7,5 | 2 | 50 | 5 | 20 | 35 | 35 | 20 | 75 | 10 |
| Trichoderma viride | 200 | 200 | 50 | 75 | 50 | 50 | 50 | 100 | 100 | 50 |

Beispiel B (Fungizide Wirkung in Anstrichmitteln)

Die fungizide Wirkung in Anstrichmitteln wird durch Prüfung der Schimmelfestigkeit der mit den Anstrichmitteln erhaltenen Anstriche bestimmt.

Die Prüfung wird in Anlehnung an den Report 219 der Defense Standards Laboratories Maribyrnong/Australien wie folgt durchgeführt:

Das zu prüfende Anstrichmittel wird beidseitig auf eine geeignete Unterlage gestrichen.

Um praxisnahe Ergebnisse zu erhalten wird ein Teil der Prüflinge vor dem Test auf Schimmelfestigkeit mit einem warmen Frischluftstrom behandelt (7 Tage; 40° C).

Die so vorbereiteten Prüflinge werden auf einen Agar-Nährboden gelegt. Prüfling und Nährboden werden mit Pilzsporen kontaminiert. Nach 1- bis 3-wöchiger Lagerung bei 29 ± 1 °C und 80 bis 90 % rel. Luftfeuchte wird abgemustert. Der Anstrich ist dauerhaft schimmelfest, wenn der Prüfling pilzfrei bleibt oder höchstens einen geringen Randbefall erkennen läßt.

Zur Kontamination werden Pilzsporen folgender neun Schimmelpilze verwendet, die als Anstrichzerstörer bekannt sind oder häufig auf Anstrichen angetroffen werden:

1. Alternaria tenuis
2. Aspergillus flavus
3. Aspergillus niger
4. Aspergillus ustus
5. Cladosporium herbarum
6. Paecilomyces variotii
7. Penicillium citrinum
8. Aureobasidium pullulans
9. Stachybotrys atra Corda

In Proben einer handelsüblichen Dispersionsfarbe auf Polyvinylacetatbasis wurden 0 bis 2 Gew.-%, bezogen auf den Gesamtfeststoffgehalt der Farbe, der auf ihre fungizide Wirksamkeit zu prüfenden Verbindung eingearbeitet. Bei diesem Test lieferten bereits die Proben, die nur 0,3 Gew.-%, bezogen auf Gesamtfeststoffgehalt der Farbe, des in Beispiel 19 beschriebenen 1-(Pyridyl-2-)-4-ethyl-5-hydroxy-pyrazols enthielten, dauerhaft schimmelfeste Anstriche.

Beispiel C

(Wirkung erfindungsgemäß fungizid ausgerüsteter Kunststoffe gegen Mikroorganismen)

Die Bestimmung der Wirkung, die die erfindungsgemäß fungizid ausgerüsteten Kunststoffe gegen Mikroorganismen (verschiedene Pilzgattungen) aufweisen, wurde gemäß der Schweizer Prüfnorm SNV No. 195 921 (von 1976; "Prüfung der antimykotischen Wirkung von ausgerüsteten Textilien und anderen Materialien mit Hilfe des Agardiffusionstestes") vorgenommen.

Für die Bestimmung wurden Prüfkörper (Scheiben; Durchmesser: 3 cm; Dicke je nach zu prüfendem Kunststoff: 250 µm bis 5 mm) der fungizid ausgerüsteten Kunststoffe und -zur Kontrolle - der entsprechenden nicht ausgerüsteten Kunststoffe auf 2-Schicht Nähragarplatten gelegt, die mit Sporen oder Hyphen des jeweiligen Testorganismus beimpft worden waren. Die Platten wurden 4 Wochen bei 29 ± 1 °C bebrütet. Anschließend wurde der Pilzbewuchs auf den Prüfkörpern und in der Kontaktzone unter den Prüfkörpern visuell bestimmt und, sofern sich eine Hemmzone unter den Prüfkörpern ausgebildet hatte, die Größe dieser Hemmzone durch Messung bestimmt.

Als Hemmzone wird die pilzfreie Zone bezeichnet, die nach folgender Formel berechnet wird:

$$H = \frac{D - d}{2},$$

in der

H = Hemmzone [mm]
D = Gesamtdurchmesser von Prüfkörper und Hemmzone [mm];
d = Durchmesser des Prüfkörpers [mm]

bedeuten.

In der nachstehenden Tabelle 5 sind die bei der Auswertung der Versuche erhaltenen Ergebnisse, Pilzbewuchs und Größe der Hemmzonen, angegeben, die bei Verwendung der in der Tabelle angegebenen Verbindungen in den ebenfalls in der Tabelle angegebenen Mengen in den ebenfalls in der Tabelle angegebenen Kunststoffen erhalten wurden.

Tabelle 5: Wirkung der fungizid ausgerüsteten Kunststoffe gegen verschiedene Testorganismen

Verwendete Testorganismen:
(a) Aspergillus niger
(b) Aspergillus terreus
(c) Chaetomium globosum
(d) Trichoderma viride
(e) Cladosporium herbarium
(f) Streptoverticilium reticulum

Bedeutung der verwendeten Abkürzungen:
H = Hemmzone [mm]
PB = Pilzbewuchs (4 Benotungen: kein, schwach, mittel, voll)

| als Fungizid verwendete Verbindung gemäß Beispiel | angewendete Menge [g/100 g Kunststoff] | (a) H/PB | (b) H/PB | (c) H/PB | (d) H/PB | (e) H/PB | (f) H/PB |
|---|---|---|---|---|---|---|---|
| 47 | 1,0/Polyvinylchlorid | 0/ mittel | 0/ schach | 0/ schwach | 0/ schwach | 0-6 kein | >25/ kein |
| 19 | 1,0/Polyvinylchlorid | 20-25/ kein | >25/ kein | >25/ kein | 0/ schwach | 12-15/ kein | 8-11/ kein |
| 18 | 1,0/Polyvinylchlorid | 0/ schwach | >25/ kein | >25/ kein | 0/ schwach | 15-18/ kein | 6-9/ kein |
| 27 | 0,5/Polyvinylchlorid | 3-5/ kein | 1-2/ kein | 1-2/ kein | 0/ mittel | 1-3/ kein | 8-10/ kein |
| Kontrolle | 0/Polyvinylchlorid | 0/ voll | 0/ voll | 0/ voll | 0/ voll | 0/ voll | 0/ voll |
| 19 | 0,15/Polyurethan | 4-6/ kein | 2-4/ kein | 0-1/ kein | 10-12/ kein | 8-10/ kein | 15-20/ kein |
| Kontrolle | 0/Polyurethan | 0/ voll | 0/ voll | 0/ voll | 0/ voll | 0/ voll | 0/ voll |

Polyvinylchlorid = Pasten-PVC (Emulsions-PVC)

Polyurethan = Polyester-Polyurethan (Bayflex®)

## Patentansprüche

**1.** Verwendung von 1-[Pyri(mi)dyl-(2)]-5-hydroxy-pyrazolen der Formel

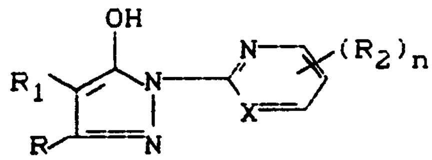

(I),

in der

- R für Wasserstoff, oder eine gegebenenfalls substituierte Alkyl-, Aralkyl- oder Alkoxygruppe steht,
- $R_1$ Wasserstoff, eine gegebenenfalls substituierte Alkyl-, Alkenyl-, Alkinyl-, Cycloalkyl-, Aralkyl-, Alkoxy-, Alkylmercapto-, Aralkoxy-, Aralkylmercapto-, Aryloxy-, Arylmercapto-, Alkoxycarbonyl-oder Aminocarbonylgruppe steht, oder $R_1$ zusammen mit R einen zweiwertigen, 3 bis 6 C-Atome in der Kette aufweisenden Alk(en)ylen-Rest bildet,
- $R_2$ für Halogen, Nitro, Cyan oder eine gegebenenfalls substituierte Alkyl-, Alkoxy-, Alkylmercapto-, Alkoxycarbonyl- oder Aminocarbonylgruppe steht,
- n Null oder eine ganze Zahl von 1 bis 3 bedeutet, wobei $R_2$ für gleiche oder verschiedene Reste stehen kann, wenn n 2 oder 3 ist und
- X für CH oder N steht,

als Mikrobizide zum Schutz technischer Materialien.

**2.** Verwendung gemäß Anspruch 1 von solchen 1-[Pyri(mi)dyl-(2)]-5-hydroxy-pyrazolen der Formel (I), in der

- X die unter Formel (I) angegebene Bedeutung hat,
- n Null ist und
- R für Wasserstoff, Alkyl oder Aralkyl und
- $R_1$ für Wasserstoff, gegebenenfalls substituiertes Alkyl, Cycloalkyl, Aralkyl, Alkoxy, Aralkylmercapto oder Alkoxycarbonyl stehen.

**3.** Verwendung gemäß Anspruch 1 von solchen 1-[Pyri(mi)dyl-(2)]-5-hydroxy-pyrazolen der Formel (I), in der

- X die unter Formel (I) angegebene Bedeutung hat,
- n Null ist und
- R für Wasserstoff, $C_6$-$C_{12}$-Alkyl oder Benzyl und
- $R_1$ für Wasserstoff, $C_1$-$C_8$-Alkyl, $C_5$-$C_7$-Cycloalkyl, $C_1$-$C_6$-Alkoxy, gegebenenfalls substituiertes Benzyl, $C_1$-$C_6$-Alkoxycarbonyl oder Benzylmercapto stehen.

**4.** Verwendung gemäß Anspruch 1 von solchen 1-[Pyri(mi)dyl-(2)]-5-hydroxy-pyrazolen der Formel (I), in der

- X für CH steht,
- n Null ist und
- R Wasserstoff und
- $R_1$ $C_1$-$C_4$-Alkyl, Benzyl, Chlorbenzyl oder Ethoxycarbonyl stehen.

**5.** Verwendung nach einem der Ansprüche 1 bis 4, dadurch gekennzeichnet, daß die technischen Materialien Anstrichmittel, Kunststoffe oder Kunststoffartikel sind.

**6.** Mikrobizide Mittel für den Materialschutz enthaltend als Wirkstoffe 1-[Pyri(mi)dyl-(2)]-5-hydroxy-pyrazolen der Formel

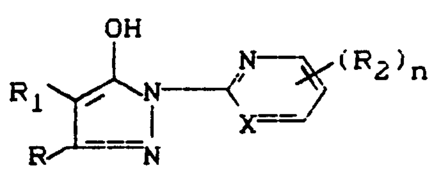

(I),

in der

R für Wasserstoff, oder eine gegebenenfalls substituierte Alkyl-, Aralkyl- oder Alkoxygruppe steht,

$R_1$ Wasserstoff, eine gegebenenfalls substituierte Alkyl-, Alkenyl-, Alkinyl-, Cycloalkyl-, Aralkyl-, Alkoxy-, Alkylmercapto-, Aralkoxy-, Aralkylmercapto-, Aryloxy-, Arylmercapto-, Alkoxycarbonyl-oder Aminocarbonylgruppe steht, oder $R_1$ zusammen mit R einen zweiwertigen 3 bis 6 C-Atome in der Kette aufweisenden Alk(en)ylen-Rest bildet,

$R_2$ für Halogen, Nitro, Cyan oder eine gegebenenfalls substituierte Alkyl-, Alkoxy-, Alkylmercapto-, Alkoxycarbonyl- oder Aminocarbonylgruppe steht,

n Null oder eine ganze Zahl von 1 bis 3 bedeutet, wobei $R_2$ für gleiche oder verschiedene Reste stehen kann, wenn n 2 oder 3 ist und

X für CH oder N steht.

**7.** 1-[Pyri(mi)dyl]-5-hyroxy-pyrazole der Formel

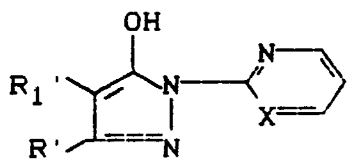

(II),

in der

X für CH oder N steht,

R' für Wasserstoff, oder eine gegebenenfalls substituierte $C_6$-$C_{10}$-Alkyl-, Aralkyl- oder Alkoxygruppe steht,

$R_1$' Wasserstoff, eine gegebenenfalls substituierte $C_6$-$C_{12}$-Alkyl-, Alkenyl-, Alkinyl-, Cycloalkyl-, Aralkyl-, Alkoxy-, Alkylmercapto-, Aralkoxy-, Aralkylmercapto-, Aryloxy-, Arylmercapto-, Alkoxycarbonyl-oder Aminocarbonylgruppe steht, oder $R'_1$ zusammen mit R' einen zweiwertigen 3 bis 6 C-Atome in der Kette aufweisenden Alk(en)ylen-Rest bildet,

mit der Maßgabe, daß R' und $R_1$' nicht gleichzeitig für Wasserstoff stehen.

**8.** 1-[Pyri(mi)dyl]-5-hydroxy-pyrazole nach Anspruch 7, dadurch gekennzeichnet, daß in Formel (II)

R' für Wasserstoff oder eine gegebenenfalls substituierte $C_6$-$C_{10}$-Alkyl, Benzyl oder $C_1$-$C_4$-Alkoxygruppe und

$R'_1$ für Wasserstoff, eine gegebenenfalls substituierte $C_6$-$C_{12}$-Alkyl, Cycloalkyl, Benzyl, $C_1$-$C_4$-Alkoxy, $C_1$-$C_4$-Alkylmercapto-, Benzyloxy, Benzylmercapto-, Phenoxy-, Phenylmercapto-$C_1$-$C_4$-Alkoxycarbonyl- oder Aminocarbonylgruppe

stehen.

Europäisches Patentamt

# EUROPÄISCHER RECHERCHENBERICHT

Nummer der Anmeldung

**EP 91 11 1537**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-3 917 469 (BAYER AG)<br>* Patentansprüche * | 7,8 | A 01 N 43/56<br>C 07 D 401/04<br>C 07 D 403/04 |
| A | CHEMICAL ABSTRACTS, Band 114, Nr. 11, 18. März 1991, Seite 73, Zusammenfassung Nr. 95150h, Columbus, Ohio, US;<br>& JP-A-02 225 469 (T. HAGA et al.) 07-09-1990 | | |
| A | EP-A-0 165 448 (BAYER AG)<br>& US-A-4 663 327 (Kat. D) | | |
| D,A | CHEMICAL ABSTRACTS, Band 108, Nr. 2, 11. Januar 1988, Seite 334, Zusammenfassung Nr. 11247k, Columbus, Ohio, US;<br>& JP-A-62 149 617 (MITSUBISHI CHEMICAL INDUSTRIES CO., LTD) 03-07-1987 | | |

RECHERCHIERTE SACHGEBIETE (Int. Cl.5)

A 01 N
C 07 D

Der vorliegende Recherchenbericht wurde für alle Patentansprüche erstellt

| Recherchenort | Abschlußdatum der Recherche | Prüfer |
|---|---|---|
| Den Haag | 30 Oktober 91 | DONOVAN T.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument